# EUROPEAN PATENT SPECIFICATION

(11) **EP 1 173 192 B1**
(45) Date of publication and mention of the grant of the patent: **15.08.2018**
(21) Application number: 00916155.5
(22) Date of filing: 08.03.2000
(51) Int. Cl.: A61K 47/50

(54) **METHODS AND COMPOSITIONS FOR TARGETED DRUG DELIVERY**
VERFAHREN UND ZUSAMMENSETZUNGEN FÜR EINE GEZIELTE VERABREICHUNG VON MEDIKAMENTEN
PROCEDES ET COMPOSITIONS POUR ADMINISTRATION CIBLEE DE MEDICAMENTS

(30) Priority: 08.03.1999 US 123352 P
(43) Date of publication of application: 23.01.2002
(73) Proprietor: Graupner, Gerhart, San Diego, CA 92171 (US)
(72) Inventor: Graupner, Gerhart, San Diego, CA 92171 (US)
(74) Representative: J A Kemp
(86) International application number: PCT/US2000/006001
(87) International publication number: WO 2000/053236

(56) References cited:
- WO-A-99/07324
- FRIDEN P M: "Utilization of an endogenous cellular transport system for the delivery of therapeutics across the blood-brain barrier" JOURNAL OF CONTROLLED RELEASE, ELSEVIER SCIENCE PUBLISHERS B.V. AMSTERDAM, NL, vol. 46, no. 1-2, 5 May 1997 (1997-05-05), pages 117-128, XP004096317 ISSN: 0168-3659
- REIST ET AL.: 'Tumor-specific anti-epidermal growth factor receptor variant III monoclonal antibodies: Use of the tyramine-cellobiose radioiodination method enhances cellular retention and uptake in tumor xenografts' CANCER RESEARCH vol. 55, October 1995, pages 4375 - 4382, XP002932959
- STEIN ET AL.: 'A non-metabolizable DTPA-peptide (DPEP) approach for production of a residualizing iodine radiolabel for targeting human lung cancer xenografts' PROCEEDINGS OF THE AMERICAN SOCIETY FOR CANCER RESEARCH vol. 39, March 1998, page 380, ABSTR. NO. 2585, XP002932958
- MCAFEE ET AL.: 'Radiolabeled peptides and other ligands for receptors overexpressed in tumor cells for imaging neoplasms' NUCLEAR MEDICINE & BIOLOGY vol. 23, 1996, pages 673 - 676, XP002932957
- LAM ET AL.: 'Targeted therapy for lymphoma with peptides lymphoma' vol. 11, no. 5, October 1997, pages 1007 - 1019, XP002932956
- FISCHMAN ET AL.: 'A ticket to ride: peptide radiopharmaceuticals' JOURNAL OF NUCLEAR MEDICINE vol. 34, no. 12, December 1993, pages 2253 - 2263, XP002932955
- REUBI J.C.: 'Neuropeptide receptors in health and disease: The molecular basis for in vivo imaging' JOURNAL OF NUCLEAR MEDICINE vol. 36, no. 10, October 1995, pages 1825 - 1835, XP002932954
- LIU ET AL.: '(99m)TC labeling of highly potent small peptides' BIOCONJUGATE CHEMISTRY vol. 8, no. 5, July 1997 - August 1997, pages 621 - 636, XP002932953

## Description

### Field of The Invention

The field of the invention is targeted drug delivery.

### Background of The Invention

Despite a wide variety of drugs, treatment of many diseases still remains problematic due to insufficient specificity of some drugs towards targeted diseased cells. Problems with specificity may further be aggravated by limited transport of the drugs across the cell membrane into the diseased cell. In some cases, drugs will only be active in a particular subcellular location. Several approaches are known in the art to improve drug delivery into a cell.

In one approach, target cells may be subjected to electroporation, during which relatively small pores in biological membranes are created for typically a few milliseconds, thereby allowing an unrestricted influx of molecules into the cell. Electroporation advantageously permits import of drugs into a cell independent of a particular chemical class and/or molecular size. However, electroporation tends to be difficult under *in vivo* conditions, and is not specific towards a particular cell type. Furthermore, electroporation has inherently a relatively high "kill rate" by disrupting organized membrane structures of the treated cells.

In another approach, fusion constructs between a drug and an importer protein are synthesized to increase the uptake of a molecule into a target cell. For example, *Prochiantz* describes the construction of a protein coupled to a homeodomain or homeopeptide derived from a Drosophila transcription factor to translocate peptides across biologic membranes [Prochiantz A. Ann N Y Acad Sci 1999;886:172-9*.* Homeodomain-derived peptides. In and out of the cells], and *Prochiantz* further suggests that the internalization of homeodomains and their fusion constructs into a cell is achieved via formation of inverted micelles. Formation of inverted micelles, however, is generally non-specific towards a particular cell type. Moreover, *Prochiantz's* fusion constructs may be immunogenic and therefore potentially limiting to an *in vitro* environment only. In another example, *Loregian et al*. describe an intracellular peptide delivery system capable of targeting specific cellular compartments [Loregian A, Papini E, Satin B, Marsden HS, Hirst TR, Palu G. Proc Natl Acad Sci USA 1999 Apr 27; 96(9): 5221-6*.* Intranuclear delivery of an antiviral peptide mediated by the B subunit of Escherichia coli heat-labile enterotoxin]. Although *Loregian's* chimeric protein is imported into a desired subcellular localization, the delivery is generally not specific towards a particular cell type. Moreover, *Loregian* suggests that the chimeric protein entered through endosomal acidic compartments, a pathway known to endosomal escape and degradation of imported molecules.

To circumvent at least some of the problems associated with degradation, and to increase the specificity of delivery, drugs may be included into a particle. For example, *Tachibana et al.* report [Tachibana R, Harashima H, Shono M, Azumano M, Niwa M, Futaki S, Kiwada H. Biochem Biophys Res Commun 1998 Oct 20;251(2):538-44*.* Intracellular regulation of macromolecules using pH-sensitive liposomes and nuclear localization signal: qualitative and quantitative evaluation of intracellular trafficking] a strategy to target macromolecules to the nucleus via the endocytic pathway. In *Tachibana's* approach, pH sensitive liposomes carry a nuclear translocation signal to direct the liposome to the nucleus. Using pH sensitive liposomes is especially desirable because various problems associated with the endocytotic pathway (*e.g.,* escape, or degradation) are typically circumvented. However, endocytotic transfer is generally not specific towards a particular cell type, thereby limiting *Tachibana's* approach.

In another approach, [Takle GB, Thierry AR, Flynn SM, Peng B, White L, Devonish W, Galbraith RA, Goldberg AR, George ST. Antisense Nucleic Acid Drug Dev 1997 Jun;7(3):177-85*.* Delivery of oligoribonucleotides to human hepatoma cells using cationic lipid particles conjugated to ferric protoporphyrin IX (heme)] *Takle et al.* report an increased target specificity of antisense RNA carrying particles to hepatocytes by packaging the antisense RNA into cationic lipid particles that are loaded with ferric protoporphyrin IX. Despite the cell-specific delivery of antisense RNA to a desired population of target cells, *Takle et al.* did not specifically deliver the antisense RNA to the nucleus, thereby significantly reducing the efficacy of antisense delivery due to cytoplasmatic nucleases and severely limited net concentration of antisense RNA in the nucleus.

In yet another approach, antibodies or antibody fragments are coupled to a drug. The use of antibodies is generally advantageous, because antibodies raised against a particular antigen on the surface of a cell exhibit a high specificity *in vitro* and *in vivo.* For example, *Chen et al.* describe an anti-EGF antibody-poly-L-lysine conjugate to form a gene delivery vehicle [Chen J, Gamou S, Takayanagi A, Ohtake Y, Ohtsubo M, Shimizu N. Cancer Gene Ther 1998; 5 (6): 357-64 Receptor-mediated gene delivery using the Fab fragments of antiepidermal growth factor receptor antibodies: improved immunogene approach]. Chen's conjugates selectively delivered a gene to a target cell via (EGFR)-mediated endocytosis, however, the transfer efficiency was only 2% with Fab fragments, and less than 0.1% when whole antibodies were employed. Moreover, antibodies are high affinity binding partners for their respective antigens and will therefore tend to almost irreversibly bind to the antigen of the cell, thereby limiting the efficacy of import to the number of available antigens.

Although various compositions and methods are known in the art to import molecules into a target cell, all or almost all of them suffer from one or more disadvantages. Therefore, there is still a need for improved methods and compositions to specifically deliver drugs to a cell compartment in a target cell.

### Summary of the Invention

The present invention is directed to a drug delivery molecule that has a targeting moiety, a routing moiety and a bioactive molecule, wherein the targeting moiety significantly binds to a receptor on the surface of a cell. Binding of the targeting moiety to the receptor (1) does not elicit a significant agonistic effect, and (2) results in an uptake of the drug delivery molecule into the cell. While the routing moiety is coupled to the bioactive molecule, the targeting moiety is coupled either to the routing moiety or to the bioactive molecule.

The present invention provides a drug delivery molecule, comprising:
a targeting moiety that significantly binds to a receptor on a surface of a cell, and wherein binding of the targeting moiety to the receptor results in an uptake of the drug delivery molecule into the cell;
a routing moiety selected from a nuclear translocation signal sequence, a cytoplasmatic retention signal, an endoplasmatic reticulum export signal or a mitochondrial translocation signal; and
a bioactive molecule, coupled to the routing moiety, wherein at least one of the routing moiety and the bioactive molecule is coupled to the targeting moiety;
   and wherein the bioactive molecule (BAM) comprises a drug or prodrug said BAM further comprising one out of a short peptide sequence that is a substrate for an intracellular protease, a disulfide bond, a charge-complementary coupling, an ionic/coordinating interaction, a hydrogen bond, a hydrophobic interaction; or the BAM is a non-drug, non-prodrug reporter group that can be used for diagnostic purposes;
   and wherein the targeting moiety
   (i) comprises a somatostatin analog that significantly binds to a polypeptide hormone receptor on a surface of a cell; or
   (ii) comprises a natural or synthetic fragment of the natural ligand of a polypeptide receptor or is a molecule homologous to a portion of the natural ligand, and significantly binds to the polypeptide receptor on a surface of a cell wherein the polypeptide receptor is a neurotransmitter receptor or a hormone receptor or a growth factor receptor.

In one aspect of the inventive subject matter, the receptor is a polypeptide hormone receptor, preferably a somatostatin type II receptor, which is located on a cell. Contemplated cells are endothelial cells proximal to a tumor, and preferred tumors include a lymphoma, mammacarcinoma, an adenocarcinoma, a glioblastoma, and a neuroblastoma.

In another aspect of the inventive subject matter, the targeting moiety is a native or synthetic fragment of the natural ligand of the receptor, or a molecule homologous to a portion of the natural ligand. Preferred targeting moieties comprise a cyclic peptide. Contemplated routing moieties include a cytoplasmatic retention signal, an endoplasmatic reticulum export signal, a mitochondrial import signal, a nuclear translocation signal, and are preferably coupled to the targeting moiety. It is also contemplated that the coupling is reversible once the drug delivery molecule has entered the cell. The bioactive molecule preferably comprises a drug or prodrug, wherein contemplated drugs may hybridize with a nucleic acid or be a nucleic acid that is expressed in a cell, interfere with (*i.e.,* inhibit or activate) a detoxification process, replication of a cell, inhibit an enzyme or metabolic pathway, or induce apoptosis.

The present invention also provides a drug delivery molecule comprising an antibody, a routing moiety, and a pharmacologically active substance,
wherein the antibody specifically binds to a somatostatin type II receptor, and is mono- or bispecific and conjugated with the pharmacologically active substance,
wherein the routing moiety is selected from a nuclear translocation sequence, a cytoplasmic retention signal, an endoplasmatic reticulum export signal or a mitochondrial translocation signal.

In a further aspect of the inventive disclosure, a method of selectively targeting an endothelial cell proximally located to an anomalous cell has a step in which it is recognized that the endothelial cell proximally located to the anomalous cell has a detectable amount of a somatostatin type II receptor, and that an endothelial cell not proximally located to the anomalous cell is devoid of the detectable amount of the somatostatin type II receptor. In a further step, the endothelial cell proximally located to the anomalous cell is presented with a compound that specifically binds to the somatostatin type II receptor.

Contemplated anomalous cells include neoplastic cells, and especially contemplated anomalous cells are mamma carcinoma cell, a prostate carcinoma cell, and a lung carcinoma cell. Other contemplated cells include ischemically stressed cells and particularly contemplated ischemically stressed cells are retinal pigment epithelial cells, myocytes, cells of the blood brain barrier, and transplanted heterologous cells.

Contemplated compounds comprise a mono-or bi-specific antibody, which may further be coupled to a pharmacological substance, including a thrombin, a radionuclide, or a growth factor, a cytokine, and a nucleic acid. Further contemplated compounds may comprise somatostatin, a somatostatin analog, a fluorophor, a chromophore, a chromogenic substrate, a nucleic acid, and a hapten for a secondary antibody.

In yet other aspects, the presence of detectable amounts of a somatostatin type II receptor on an endothelial cell proximal to a tissue is correlated with a disease of the tissue. In subsequent steps, compounds for administration to the endothelial cell specifically bind to the somatostatin type II receptor.

Various objects, features, aspects and advantages of the present invention will become more apparent from the following detailed description of preferred embodiments of the invention, along with the accompanying drawing.

### Brief Description of The Drawing

Fig. 1A-D are exemplary structures of drug delivery molecules according to the inventive subject matter.
Fig. 2A-E are microphotographs of cells incubated with an exemplary drug delivery molecule according to the inventive subject matter.
Fig. 3 is a graph showing fluorescence intensity in target cells and in control cells incubated with drug delivery molecules according to the inventive subject matter.
Fig. 4 is an immuno-electromicrograph of controls and of choroidal endothelial cells having detectable amounts of somatostatin type II receptors.

### Detailed Description

As used herein, the term "receptor" refers to a structure on the surface of a cell that is at least partially accessible from the outside of the cell, wherein the structure is capable of binding another molecule with high specificity, and wherein binding of the other molecule modulates directly or indirectly a physiological function of the cell by a mechanism other than an increase of a metabolite. The contemplated structure may thereby be a single molecule (typically a polypeptide) with a single binding site, or a multi-component structure in which a plurality of components form a binding site, or in which at least one of the components has a single binding site (*e.g.,* a receptor including modulatory polypeptides without binding function to the other molecule). While contemplated receptors typically have a high binding specificity towards a single molecule, other contemplated receptors may be specific for more than one molecule. For example, a receptor in a neurohormonal signaling pathway may be a multi-component receptor having at least a portion with a hormone binding domain outside of the cell and another portion within the cell coupled to the first portion (*e.g.,* a G-protein) that produces a second signal (*e.g.,* cGMP). It should be especially recognized that a transporter on a cell membrane (*e.g.* a glucose transporter) is not regarded as a receptor under the scope of this definition, because a glucose transporter modulates a physiological function (*e.g.,* glycolysis) by an increase of a metabolite.

As also used herein, a molecule that is not identical with the physiological (*i.e.* natural) ligand of a receptor of a cell has a "significant agonistic effect", when binding of the molecule to the receptor elicits a measurable effect on the cell that is at least 25% of a measurable effect of the physiological ligand under comparable conditions. As further used herein, the term "significant binding" means non-covalent binding of two molecules to each other with a dissociation constant K_{D} of less than about 1x10⁻⁴mol⁻¹. As still further used herein, the term "uptake" refers to any mode of internalization of a molecule into a cell, including receptor-mediated endocytosis, internalization of a receptor-ligand complex, etc.

In **Structures 1A** and **1B,** general structures of contemplated drug delivery molecules are shown and include a targeting moiety TM, a routing moiety RM, and a bioactive molecule BAM. 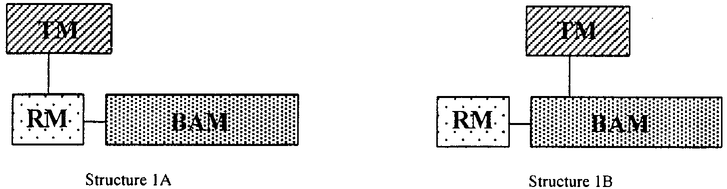 Contemplated targeting moieties bind significantly to a receptor on a surface of a cell, wherein binding of the targeting moiety to the receptor does not elicit a significant agonistic effect, and wherein binding of the targeting moiety to the receptor results in an uptake of the drug delivery molecule into the cell. A routing moiety is coupled to a bioactive molecule, wherein at least one of the routing moiety and the bioactive molecule is coupled to the targeting moiety.

In a preferred aspect of the inventive subject matter, the targeting moiety is a fragment of somatostatin that has no significant agonistic effect. Further preferred targeting moieties include synthetic cyclic or non-cyclic peptides that specifically bind to a somatostatin type II receptor and examples of preferred targeting moieties having no significant agonistic effect are shown in **Structures 2A-D.**

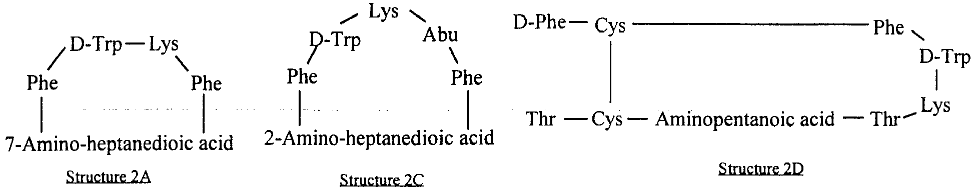

D-Phe-Cys-Phe-D-Trp-Orn-Abu-Cys-Thr Structure 2B

Although preferred targeting moieties comprise a fragment or portion of the natural ligand of a receptor (e.g., the somatostatin type II receptor), alternative targeting moieties need not be restricted to portions of the natural ligands of a receptor, and appropriate targeting moieties may include a wide variety of peptides. For example, alternative targeting moieties may include peptides that are homologous to a portion of the natural ligand. Homologous portions may be especially advantageous, where cross-binding with related receptors need to be reduced. Furthermore, where solubility and/or stability of contemplated targeting moieties is a concern, non-proteinogenic amino acids may be included into the targeting moiety. Antibodies, antibody fragments, and antibody-like proteins are not considered a targeting moiety under the scope of this definition.

It should further be appreciated that alternative targeting moieties may also comprise organic and/or organo-metallic compounds, so long as alternative targeting moieties (1) significantly bind to a receptor, (2) binding does not elicit a significant agonistic effect, and (3) binding of the targeting moiety to the receptor results in an uptake of the targeting moiety. Organic and/or organo-metallic compounds may be especially desirable to reduce potential immunogenicity or structural instability while retaining affinity to the target receptor.

Regardless of the chemical composition of contemplated targeting moieties, it is particularly preferred that appropriate targeting moieties include fragments of somatostatin and/or somatostatin analogs which are known to lack agonistic effect. Fragments and analogs lacking an agonistic effect are known in the art [Barrie, R., et al. J. Surg. Res. 1993, 55(4):446-450. Inhibition of Angiogenesis by Somatostatin and Somatostatin-like compounds is structurally dependent; Coy et al., U.S. Pat. No. 4,508,711]. It is even more preferred that appropriate targeting moieties include fragments of somatostatin and/or somatostatin analogs that are known to lack agonistic and antagonistic effect.

It should further be appreciated that the inventive subject matter presented herein is not restricted to a particular type of receptor, and contemplated receptors include hormone receptors, growth factor receptors, neurotransmitter receptors, etc. Especially contemplated receptors include polypeptide hormone receptors, and most preferably a somatostatin type II receptor. Moreover, contemplated receptors need not be limited to a particular structure. Therefore, appropriate receptors may comprise single-subunit and multi-subunit receptors, wherein at least one of the subunits has a binding site or wherein at least two of the subunits form a binding site.

It is preferred that target cells include a microvascular endothelial cell proximally located to a tumor, and particularly contemplated tumors include a mammacarcinoma, an adenocarcinoma, a sarcoma, a lymphoma, a glioblastoma, and a neuroblastoma. The term "endothelial cell proximally located" to a tumor, cell, or tissue refers to an endothelial cell that surrounds, infiltrates or is present within the tumor, cell or tissue. However, in alternative aspects, many target cells other than a microvascular endothelial cell proximally located to a tumor are also contemplated, so long as the cell has a receptor on the surface of the cell that binds specifically to the targeting moiety, and particularly contemplated cells include those that significantly express somatostatin type II receptor on their cell surface, such as pancreatic adenocarcinoma parenchyma and neuroblastoma parenchymatic cells.

In another preferred aspect of the inventive subject matter, the routing moiety comprises a nuclear translocation signal sequence (*e.g.,* KKLK), however, various alternative routing sequences are also contemplated, including a cytoplasmatic retention signal, an endoplasmatic reticulum export signal, and a mitochondrial translocation signal. Where the routing moiety and the targeting moiety are coupled together it is especially contemplated that a carboxyl group from an amino acid of the targeting moiety forms an amide bond with an amino group from an amino acid (*e.g*., ε-amino group from a Lys) of the routing moiety. It should be appreciated, however, that many alternative couplings are also contemplated, including covalent and non-covalent coupling. For example, where limited stability of the coupling between the routing moiety and the targeting moiety is desirable, one or more disulfide bonds are contemplated. Disulfide bonds are particularly advantageous, because disulfide bonds are typically stable in an extracellular environment, but readily cleaved within a cell. In another example, a coupling includes a short peptide sequence that is a substrate for an intracellular protease. Such a substrate sequence will specifically be cleaved within a cell and is therefore especially useful in applications where the routing moiety need to be removed from the targeting moiety within the cell. Other non-covalent coupling may include non-peptide couplings, which may be polymers (*e.g.,* functionalized polyethylene glycol), or short bifunctional crosslinker-type molecules (*e.g.,* bismaleimide).

Non-covalent coupling may be particularly advantageous where the stability of the coupling is dependent on the solvent, or other microenvironmental conditions. For example, non-covalent coupling may include charge-complementary coupling (*e.g.,* poly-lysine/poly-glutamate), hydrophobic interaction (*e.g.* leucine zipper), hydrogen bonding (*e.g.,* complementary nucleic acids), or ionic/coordinating interactions (Ni-His complexation), etc.

In a further preferred aspect of the inventive subject matter, the bioactive molecule may comprise a drug or a prodrug, and the term "bioactive molecule" as used herein generally refers to any compound that has a substantial effect on the functioning of a cell, whether as a result of its chemical composition and/or as a result of its isotopic composition. Contemplated bioactive molecules include drugs and prodrugs, and particularly contemplated drugs include a molecule that hybridizes with a nucleic acid, or that interferes with a detoxification process in a cell. Other particularly contemplated drugs inhibit an enzyme, replication of a cell, or induce/prevent apoptosis. For example, molecules hybridizing with a nucleic acid may include sense and antisense DNA, RNA, PNA, and their chemical analogs. Molecules interfering with a detoxification process may include inhibitors of transporters involved in multi-drug resistance. Enzyme inhibitors may include suicide inhibitors, competitive, non-competitive and allosteric inhibitors of hydrolases, ligases, lyases, etc. Furthermore, many drugs are known to inhibit replication of a cell (*e.g.,* α-amanitin) or induce apoptosis (*e.g.,* Fas ligand) all of which are incorporated herein by reference.

In further alternative aspects, the bioactive molecule may also be a non-drug, non-prodrug reporter group that can be used for diagnostic purposes, and contemplated reporter groups include a fluorophor, a radionuclide, a chromophore, and a chromogenic substrate.

With respect to the coupling of the bioactive molecule to the targeting moiety it should be appreciated that the same considerations as for the coupling between the targeting moiety and the routing moiety apply. Furthermore, while it is preferred that the coupling is either between the targeting moiety and the routing moiety, or the targeting moiety and the bioactive molecule, double coupling of the targeting moiety to routing moiety and the bioactive molecule is also contemplated.

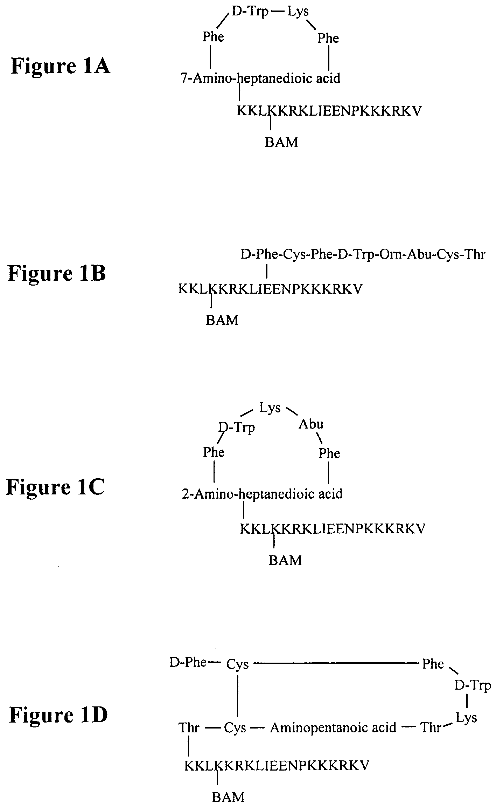

It should especially be appreciated that drug delivery molecules according to the inventive subject matter may be employed to deliver a particular drug with high cell and cell compartment specificity. Moreover, contemplated molecules may be utilized to deliver a particular drug specifically to cells that are associated with diseases, in cases where the disease is coupled the presence or overexpression of the receptor being targeted by the targeting moiety. For example, drug delivery molecules according to the inventive subject matter may be employed to inhibit angiogenesis by targeting the somatostatin type II receptor in a drug delivery molecule having a nuclear targeting sequence coupled to an antisense nucleic acid that blocks expression of a vital gene. **Figures 1A-D** show exemplary structures of drug delivery molecules according to the inventive subject matter, wherein BAM is a bioactive molecule.

Contemplated administrations of drug delivery molecules according to the inventive subject matter include *in vivo* and *in vitro* applications of the drug delivery molecule to a target cell. For example, where a particular population of cells is cultured *ex vivo,* the culture medium may be supplemented with the drug delivery molecule. Alternatively, cells may be transiently or permanently transfected with a recombinant nucleic acid to express a target receptor. Such recombinant cells may then be subjected to incubation with the drug delivery molecule. *In vivo* applications may include intravenous injection, oral administration, transdermal application, etc.

It should further be appreciated that preferred drug delivery molecules do not have a significant agonistic effect, and in particularly preferred aspects, the drug delivery molecules do not have a significant agonistic and antagonistic effect due to the particular structure of the targeting moiety.

It should especially be recognized that targeting the somatostatin type II receptor is particularly advantageous because the inventor discovered that endothelial cells that are not proximally located to an anomalous cell do not have detectable amounts of somatostatin type II receptors, whereas endothelial cells that are proximally located to an anomalous cell have detectable amounts of somatostatin type II receptors. As used herein, the term "detectable amounts" refers to amounts of receptors that can be detected by current protocols for immunofluorescence or immuno-electron microscopy.

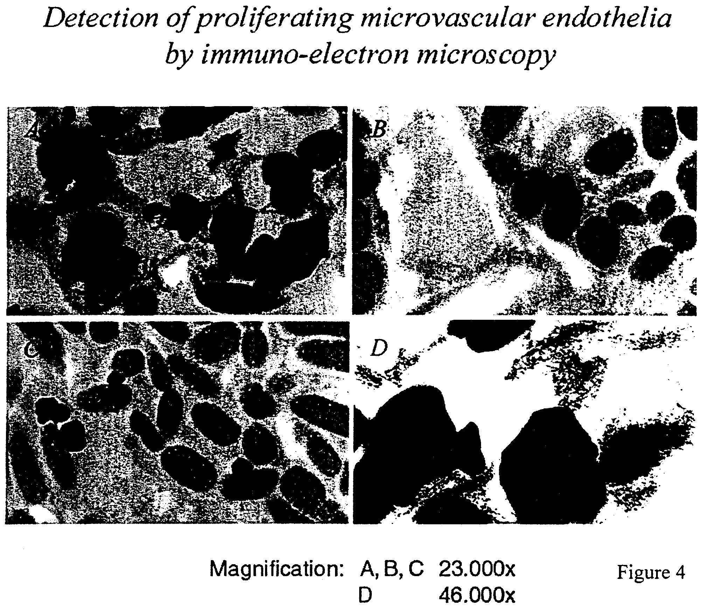

An example for this discovery is illustrated in Figures 4A-D, showing detection of somatostatin type II receptors in activated microvascular endothelia by silver-enhanced immunogold reactivity in a rat model for laser-induced macular degeneration. Endothelial cells are proximally located within a population of anomalous cells in macular degeneration foci. **Figure 4A** depicts specific antibody staining of choroidal endothelia in a region of neovascularization (anomalous cells are located in laser-treated experimental tissue). Large fusion silver particles indicate clustered gold particles in high density on the luminal surface of endothelial cells, but not on the abluminal surface of endothelial cells. **Figure 4B** shows specific antibody inhibited by specific recognition peptide (laser-treated experimental tissue). No immunoreactivity in either vascular tissue or muscular tissue posterior to choroida (the latter is shown). **Figure 4C** shows specific antibody staining of muscular tissue posterior to choroida (control tissue without laser treatment). No immunoreactivity in endothelial region, but small silver particles at low density demonstrate immunostaining of somatostatinergic neurons in muscular tissue. **Figure 4D** shows a magnified image of microvascular cross-section in a region of neovascularization (same region as shown in A). Large fusion silver particle touching luminal surface of endothelial cell (nucleus, lower right area).

Consequently, it is contemplated that endothelial cells proximally located to an anomalous cell can be selectively targeted, and a contemplated method of selectively targeting an endothelial cell proximally located to an anomalous cell has the following steps: In one step, it is recognized that the endothelial cell proximally located to the anomalous cell has a detectable amount of a somatostatin type II receptor, and that an endothelial cell not proximally located to the anomalous cell is devoid of the detectable amount of the somatostatin type II receptor. In another step, the endothelial cell proximally located to the anomalous cell is presented with a compound that specifically binds to the somatostatin type II receptor.

In a preferred aspect of the inventive subject matter, the anomalous cell is a neoplastic cell, and particularly contemplated neoplastic cells include a mamma carcinoma cell, a prostate carcinoma cell, and a lung carcinoma cell. Other contemplated anomalous cells include an ischemically stressed cell, for example an ischemically stressed myocyte, cell of a blood brain barrier or a transplanted heterologous cell. However, it is generally contemplated that an "anomalous cell" is a cell that, due to a condition within the cell or within the cell's environment, expresses the somatostatin type II receptor in a detectable amount, whereas the cell would not normally express the somatostatin type II receptor in a detectable amount (*e.g.,* tumor parenchyma).

In further aspects of the inventive subject matter, it is contemplated that the compound that specifically targets the endothelial cell comprises an antibody that specifically binds to the somatostatin type II receptor, wherein the antibody may be mono-specific or bi-specific. Mono-specific or bi-specific antibodies with specificity against the somatostatin type II receptor can be produced by a variety of protocols, and all available protocols are contemplated to be useful for production of contemplated antibodies. Regardless the antibody structure, it is further contemplated that appropriate antibodies may be conjugated with a pharmacologically active substance. For example, where prevention of neovascularisation of a tumor is desirable, the pharmacological substance may be a thrombin that may precipitate clogging of the microvessel. Alternatively, the pharmacological substance may be a radionuclide to specifically direct energy of the radioactive decay of the radionuclide to a desired site (*e.g.,* a tumor). It is still further contemplated that appropriate compounds may also stimulate growth or proliferation of a microvascular endothelial cell. Thus, it is contemplated that the compound may comprise growth factors, cytokines, or a nucleic acid that can be expressed within the microvascular endothelial cell. There are many methods known in the art to conjugate substances to an antibody, and it is contemplated that such methods are appropriate for use herein.

It should further be appreciated that the method of selectively targeting the endothelial cell may be employed in a diagnostic environment. For example, the method may be employed to determine the presence of an anomalous cell in a specimen. Alternatively, the method of selectively targeting may be utilized to screen for an anomalous cell or cell population. Therefore, it is contemplated that the compound may comprise a reporter group. Many reporter groups are known in the art and it is contemplated that all of the known reporter group can be used in conjunction with the teachings presented herein. For example, where direct access to the endothelia cell is possible the reporter group may be a fluorophor, a chromophore, a hapten for a secondary antibody or a chromogenic substrate. A nucleic acid may be employed as a reporter group where high signal amplification is desired. Where direct access to the endothelial cell is not possible, the reporter group may be a radionuclide or a metal detectable in scanning.

In particularly contemplated aspects of the inventive subject matter, the compound may comprise a drug delivery molecule with a targeting moiety that significantly binds to the somatostatin type II receptor, wherein binding of the targeting moiety to the receptor does not elicit a significant agonistic effect, and wherein binding of the targeting moiety to the receptor results in an uptake of the drug delivery molecule into the cell, and wherein the drug delivery molecule further has a routing moiety and a bioactive molecule, coupled to the routing moiety, wherein at least one of the routing moiety and the bioactive molecule is coupled to the targeting moiety. Alternatively, contemplated compounds may also comprise somatostatin, or a somatostatin analog.

With respect to the step of presenting the endothelial cell proximally located to the anomalous cell with the compound, various methods are contemplated, including direct and indirect methods. Direct methods include incubation of the endothelial cell in a solution containing the compound, while indirect methods include injection of the compound into an organism harboring the endothelial cell proximally located to the anomalous cell.

Anomalous cells can be found in a variety of circumstances. For example, it is known that in some diabetic conditions peripheral circulation is restricted in some tissues due to various causes. As a consequence, malperfused tissue will contain anomalous cells, and the endothelial cells proximally located to the anomalous cells will have somatostatin type II receptors in a detectable amount. Therefore, a method of improving circulation may have one step in which a tissue having a reduced circulation is correlated with a detectable amount of a somatostatin type II receptor on an endothelial cell proximally located to the tissue having the reduced circulation. In another step, a compound is administered to the endothelial cell, wherein the compound specifically binds to the somatostatin type II receptor, and wherein the compound stimulates a cell proliferation of the endothelial cell. It is contemplated that the reduced circulation may be caused by a stenosis of a blood vessel, and it is especially contemplated that the stenosis occurs in a blood vessel located in the brain or heart.

With respect to the compound that stimulates cell proliferation of the endothelial cell it is contemplated that various drug delivery molecules as described above are appropriate so long as such drug delivery molecules have a targeting moiety that binds specific to the somatostatin type II receptor, and so long as the bioactive molecule stimulates the proliferation of the endothelial cell. For example, it is contemplated that appropriate bioactive molecules include cytokines and growth factors.

Furthermore, it is also known that in some vision disorders circulation supporting retinal tissue, and especially the macular region, is restricted due to various causes. Consequently, malperfused tissue will contain anomalous cells, and endothelial cells proximally located to the anomalous cells will have somatostatin type II receptors in a detectable amount. Therefore, a method of improving vision may have one step in which a tissue having a focus of macular degeneration is correlated with a detectable amount of a somatostatin type II receptor on an endothelial cell proximally located to the malperfused tissue. In another step, a compound is administered to the endothelial cell, wherein the compound specifically binds to the somatostatin type II receptor, and wherein the compound inhibits cell proliferation of the endothelial cell. With respect to the compound that inhibits cell proliferation of the endothelial cell, the same considerations as described above apply.

Still further, it is known that in some health conditions, and especially in neoplasms, tumors will be malperfused due to limited nutrient diffusion into the neoplastic tissue. Consequently, neoplastic tissue will contain anomalous cells, and endothelial cells proximally located to the anomalous cells will have somatostatin type II receptors in a detectable amount. Therefore, a method of improving a health condition may have one step in which a tissue having a neoplasm is correlated with a detectable amount of a somatostatin type II receptor on an endothelial cell proximally located to the tissue having the neoplasm. In another step, a compound is administered to the endothelial cell, wherein the compound specifically binds to the somatostatin type II receptor, and wherein the compound inhibits cell proliferation of the endothelial cell.

It is contemplated that the type of neoplasm is not limiting to the inventive subject matter, and appropriate neoplasms may include a lymphoma, a sarcoma, an adenocarcinoma, and a teratocarcinoma. With respect to the compound that inhibits cell proliferation of the endothelial cell, it is contemplated that various drug delivery molecules as described above are appropriate as long as such drug delivery molecules have a targeting moiety that binds specifically to the somatostatin type II receptor, and as long as the bioactive molecule inhibits the proliferation of the endothelial cell. For example, it is contemplated that appropriate bioactive molecules may include drugs and prodrugs that may inhibit an enzyme, replication, DNA and RNA synthesis, etc.

### Examples

The following examples describe synthesis and an exemplary application of a drug delivery molecule according to the inventive subject matter.

### Example 1

Compound I, Compound II, Compound III, and Compound IV were synthesized in a stepwise manner by the Fmoc solid-phase method. Fmoc amino acids, 2-(1H-Benzotriazole-1-yl)-1,1,3,3-tetramethyluronium tetrafluoroborate (TBTU), 9-Fluorenylmethoxycarbonyl-N-hydroxysuccinimide (Fmoc-OSu), Fmoc-Val-Wang resin, and H-O-*tert*-butyl-L-threonine-2-chlorotrityl resin were obtained from AnaSpec (San Jose, CA) and other reagents for peptide synthesis were from Protein Technologies, Inc. The synthesis was in a 0.025 mmol scale on a Rainin Symphony multiple peptide synthesizer with a 2-(1H-benzotriazol-1-yl)-1,1,3,3-tetramethyluronium hexafluorophosphate (HBTU)/n-methylmorpholine activation strategy. Upon completion of the synthesis, the peptides were cleaved from the resin and deprotected (side chain) with TFA containing 2.5% 1,2-ethanedithiol (EDT) and 2.5% H2O. The resin was filtered out from the reaction mixture and the peptides were precipitated with ether.

Routing peptide: KRKLIEENPKKKRKV was synthesized first and then deprotected. Lysine (dissolved in 80% DMF in H₂O) was manually added. The reaction between Texas Red-X succinimidyl ester and the unprotected lysine was allowed to proceed overnight. The rest of the peptide (KKL) was synthesized by the Fmoc solid-phase method.

Compound I: 7-amino-1-carboxy-heptanoic acid (Fmoc-Asu-OH, Fmoc-L-α-aminosuberic acid)FKdWF was synthesized, cleaved and then cyclized by adding 4 equivalents of TBTU and 8 equivalents of diisopropylethylamine (DIEA) for 4h. Cyclized peptide and routing peptide were linked by adding 4 equivalents of TBTU and 8 equivalents of diisopropylethylamine (DIEA) for 4h.

Compound II: ENPKKKRKV was synthesized first and then deprotected. Glu was manually added. The dFCFdW-Orn-Abu-CThr(ol) ClEt-2 resin was used to prepare the fully protected fragment. The EENPKKKRKV-Wang resin and fully protected fragment were joined by adding 4 equivalents of TBTU and 8 equivalents of diisopropylethylamine (DIEA) for 4h. Next, the KRKLI sequence was added to the peptide and then deprotected. Lysine (80% DMF in H2O) was manually added. The reaction between Texas Red-X succinimidyl ester and the unprotected lysine was allowed to proceed overnight. The rest of the peptide KKL was synthesized by the Fmoc solid-phase method.

Compound III: Fmoc 6-amino-1-carboxy-heptanoic acid (Fmoc-DL-2-aminoheptanedioic acid was prepared by a H₂N-CH®-COOH + Fmoc-OSu-------> Fmoc-NH-CH®-COOH + O-Su reaction. 6-amino-1-carboxy-heptanoic acid (DL-2-aminoheptanedioic acid)F-Abu-KdWF was synthesized, cleaved and then cyclized by adding 4 equivalents of TBTU and 8 equivalents of diisopropylethylamine (DIEA) for 4h. Cyclized peptide and routing peptide were linked by adding 4 equivalents of TBTU and 8 equivalents of diisopropylethylamine (DIEA) for 4h.

Compound IV: KRKLIEENPKKKRKV was synthesized first and then deprotected. Lysine (dissolved in 80% DMF in H2O) was manually added. The reaction between Texas Red-X succinimidyl ester and the unprotected lysine was allowed to proceed overnight. The rest of the peptide dFCFdWKT-aminopentanoic acid-CTKKL was synthesized by the Fmoc solid-phase method. Synthesis of disulfide-linkage cyclopeptide was done by Cys-oxidation using a dimethyl sulfoxide-mediated oxidation reaction.

### Example 2

### Cellular uptake measurements of somatostatin derivatives coupled to a nuclear routing peptide

Human neuroblastoma cells (SK-N; SSR2A-positive; (SSR2A=somatostatin type II receptor)) were grown to 50-70% confluency in 25 cm²-flasks, using DMEM medium supplemented with 1 mM Na-pyruvate, 5 mM non-essential amino acids, and 10% FCS. Hamster CHO cells (SSR2A-negative) were grown under identical conditions. For a typical experiment, cells from one flask were trypsinized, counted in a hematocytometer, seeded as suspension of 1x10⁵ cells/ml onto chamber slides (LabTek single chamber slides #177372, or double chamber slides, or quadruple chamber slides). 24h after seeding, cells were washed 2-3 times with DME and pre-incubated for 30 min with either 1µM somatostatin (Sigma, tissue culture grade) in DME (uptake control condition) or with DME (experimental condition). After removal of the pre-incubation solution, cells were incubated for 2h in either DME containing 1 µM somatostatin plus 1 µM experimental peptide (uptake control condition), or in DME containing 1µM experimental peptide only (experimental condition). Experimental peptides used were compound I, compound II, compound III, compound IV, and routing peptide.

D-Trp — Lys
Phe
Phe
7-Amino-heptanedioic acid
KKLKKRKLIEENPKKKRKV
Texas Red
Compound I

Lys
D-Trp
Abu
Phe
Phe
2-Amino-heptanedioic acid
KKLKKRKLIEENPKKKRKV
Texas Red
Compound III

D-Phe — Cys ——————————— Phe
D-Trp
D-Phe-Cys-Phe-D-Trp-Orn-Abu-Cys-Thr
Lys
Thr — Cys — Aminopentanoic acid — Thr
KKLKKRKLIEENPKKKRKV
KKLKKRKLIEENPKKKRKV
Texas Red
Compound II
Compound IV
Texas Red

After removal of the incubation solution, cells were rinsed with DME and grown in full medium for additional 30 min, 2h, 24h, 48h, or 72h. The most informative time point of nuclear uptake was determined in initial experiments. Four conditions per experimental peptide were investigated 48h after treatment: Experimental compound with or without somatostatin in neuroblastoma cells, and experimental compound with or without somatostatin in CHO cells.

At times as indicated, cells were rinsed with PBS three times and fixed in 4% paraformaldehyde/PBS overnight at 4-7°C, rinsed with PBS three times and mounted in SlowFade (Molecular Probes).

Confocal analysis was performed with a Zeiss LSM-300 microscope. For each treatment condition, pairs of images (DIC and red channel fluorescence) were taken at 100x oil immersion, with settings for fluorescent signal recording kept constant. Z-sectioning confirmed optimal choice of confocal plane. Images were displayed at identical magnification factors in grayscale mode by Adobe Photoshop software. No signal enhancement or modification procedures were used prior to the measurement of pixel intensities.

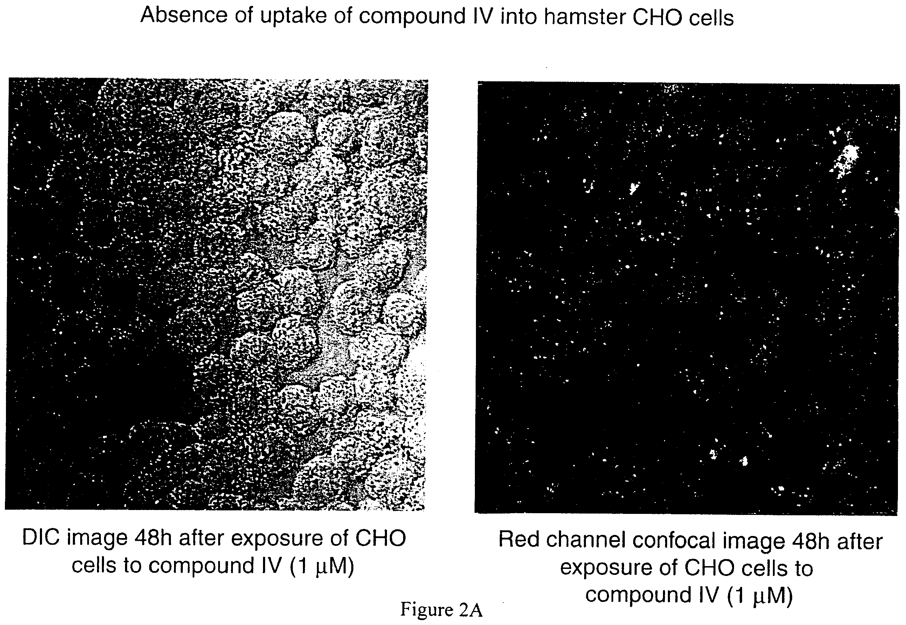

As an example, paired images recorded from cells treated with compound IV or routing peptide and grown for additional 48h are presented in Figures 2A to 2E. To evaluate peptide uptake into nuclei proper, nuclei were identified in DIC images, and matching regions in fluorescent images were scanned. Pixels displaying less than 100% black saturation were identified across the nucleus, and their values were recorded and averaged. The pixel intensity averages of at least three nuclei were used to determine the fluorescence intensity of nuclear staining at each experimental condition. Each percent of reduction in black saturation is expressed as 1 relative light unit (*i.e.* reduction to 75% black saturation corresponds to 25 relative fluorescent light units).

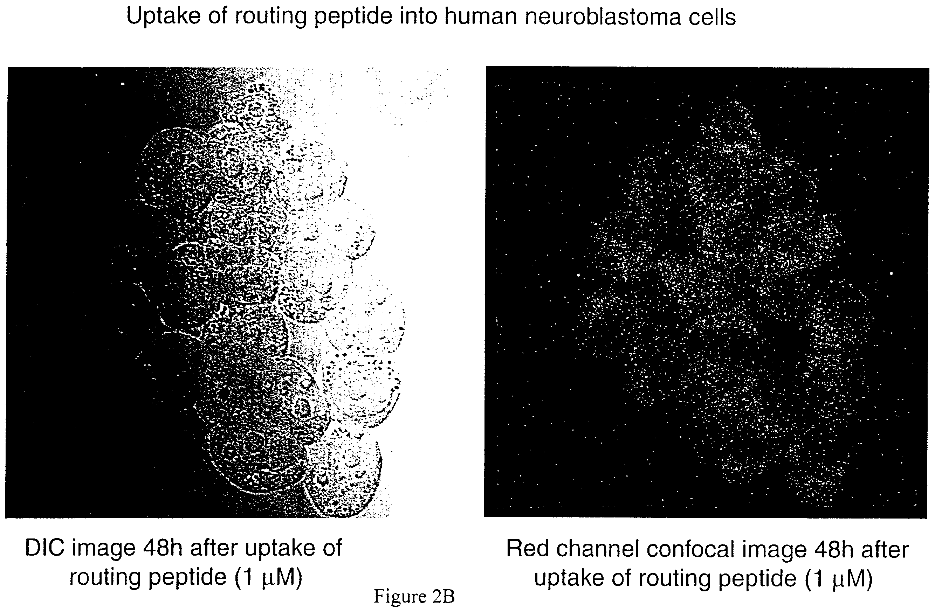

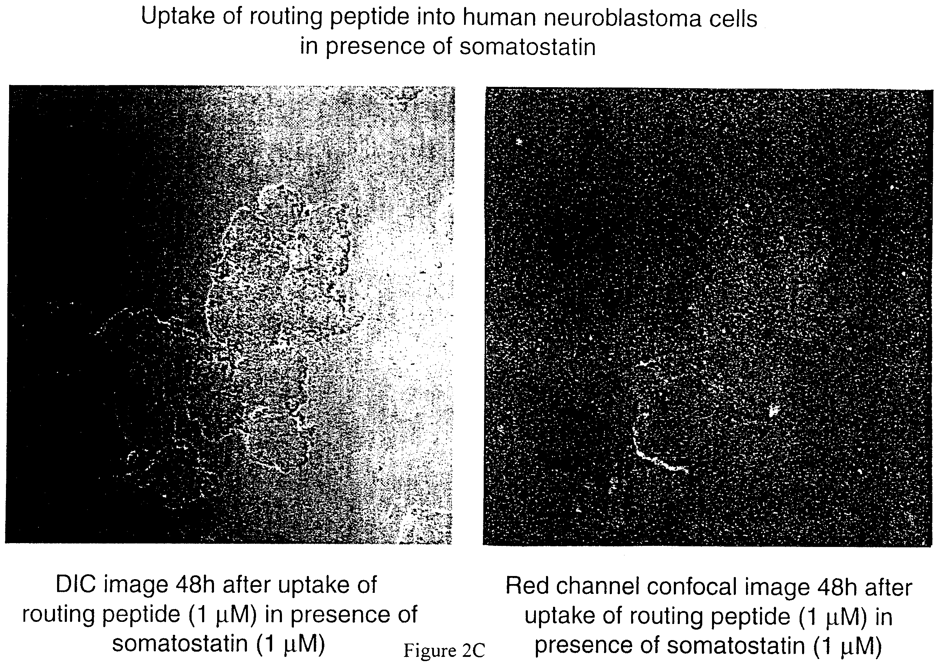

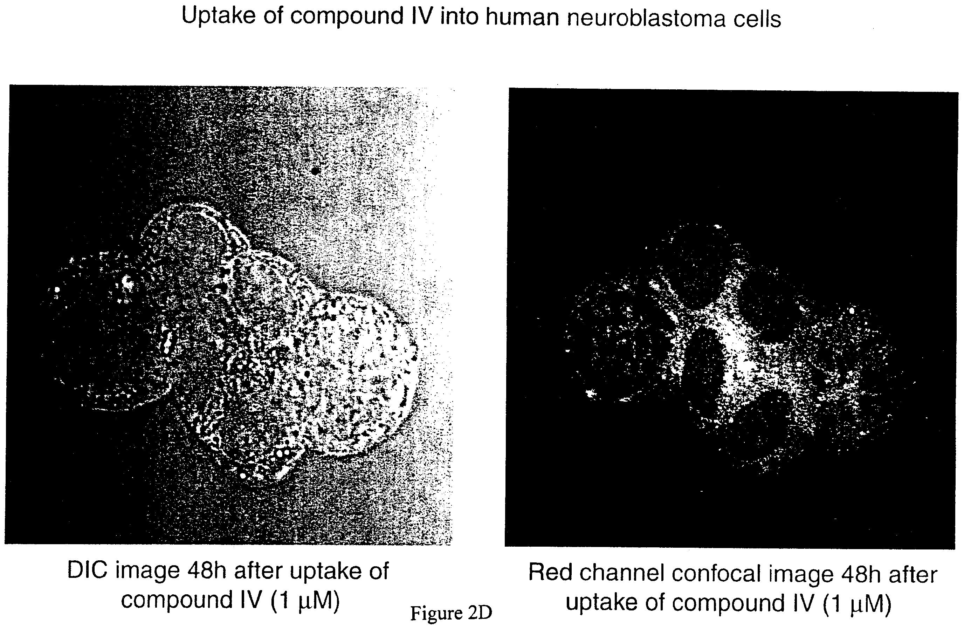

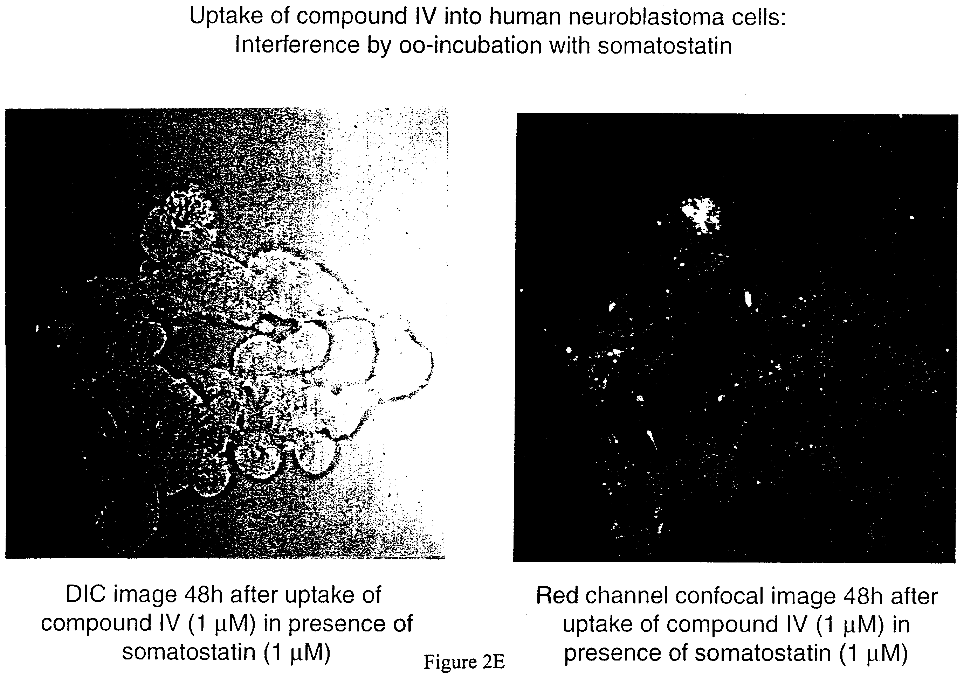

**Figure 2A** illustrates the absence of uptake of compound IV into hamster CHO cells. **Figure 2B** illustrates the absence of uptake of the routing peptide-Texas red conjugate into human neuroblastoma cells, while **Figure 2C** illustrates the absence of uptake of the routing peptide-Texas red conjugate into human neuroblastoma cells in the presence of somatostatin. **Figure 2D** shows the uptake of compound IV into the cell and nuclei of human neuroblastoma cells, and **Figure 2E** shows the uptake of compound IV into the cell and nuclei of human neuroblastoma cells in the presence of somatostatin. In all of Figures 2A-E, the left image represents the DIC photomicrograph and the right image represents the corresponding red channel confocal microphotograph of the same sample.

### Relative nuclear fluorescence intensity in human neuroblastoma cells versus hamster CHO cells 48h after treatment

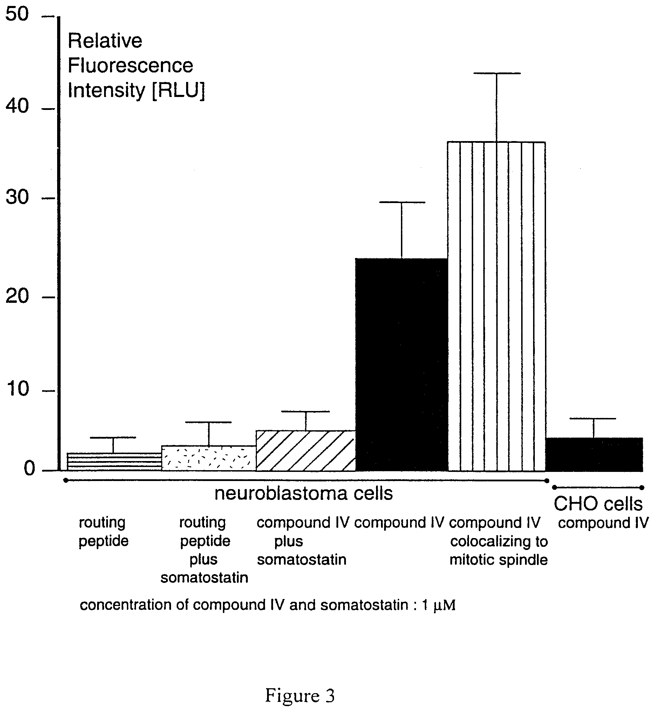

The relative nuclear fluorescence intensity in human neuroblastoma cells versus hamster CHO cells 48h after treatment is shown in **Figure 3****.** (Routing peptide (1 µM) in neuroblastoma cells (bar 1), routing peptide (1 µM) plus somatostatin (1µM) in neuroblastoma cells (bar 2), compound IV (1 µM) plus somatostatin (1 µM) in neuroblastoma cells (bar 3), compound IV (1µM) in neuroblastoma cells (bar 4), compound IV (1 µM)cο-localizing to the mitotic spindle in neuroblastoma cells (bar 5), compound IV (1 µM) in CHO cells (bar 6). Bar heights represent the mean of pixel intensities. The symmetric error bars reflect the range of variation. 1 relative fluorescence light unit corresponds to a reduction of 1% in black saturation of a standard grayscale.)

### Results

Very low level (borderline) nuclear fluorescence was found 30 min after incubation. Specific nuclear fluorescence was increased to significant levels 2h after incubation. Strong levels were detectable through 48h. No significant nuclear fluorescence was detectable 72h after incubation.

In presence of equimolar somatostatin concentrations during the treatment period, no nuclear fluorescence was detectable after 2h. Minimal fluorescence, if any, was detectable after 48h. There was, however, substantial fluorescence associated with the outer cell membrane after 2h, and detectable levels of cytoplasmic fluorescence after48h. The increase of uptake over time suggests that most likely, the very short half life of somatostatin bound to the SSR2A was not sufficient to block entry of compound IV completely; residual compound IV bound to the cellular plasma membrane could have entered the cell through the SSR2A uptake pathway after degradation of somatostatin was complete.

Routing peptide did not display significant staining of the cell surface in the absence of somatostatin; fluorescence intensity was not reduced in the presence of somatostatin. These two observations corroborate the anticipated result that routing peptide by itself has no significant affinity for SSR2A and does not enter neuroblastoma cells by means of an alternative pathway.

In CHO cells, which do not express SSR2A, no significant fluorescence of either nuclei or plasma membrane was found 48h after treatment. The presence or absence of somatostatin in the incubation medium did not affect the fluorescence intensities in CHO cells.

Thus, specific embodiments and applications of methods and compositions for targeted drug delivery have been disclosed. The terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

## Claims

1. A drug delivery molecule, comprising:
a targeting moiety that significantly binds to a receptor on a surface of a cell, and wherein binding of the targeting moiety to the receptor results in an uptake of the drug delivery molecule into the cell;
a routing moiety selected from a nuclear translocation signal sequence, a cytoplasmatic retention signal, an endoplasmatic reticulum export signal or a mitochondrial translocation signal; and
a bioactive molecule, coupled to the routing moiety, wherein at least one of the routing moiety and the bioactive molecule is coupled to the targeting moiety;
and wherein the bioactive molecule (BAM) comprises a drug or prodrug, said BAM further comprising one out of a short peptide sequence that is a substrate for an intracellular protease, a disulfide bond, a charge-complementary coupling, an ionic/coordinating interaction, a hydrogen bond, a hydrophobic interaction; or the BAM is a non-drug, non-prodrug reporter group that can be used for diagnostic purposes;
and wherein the targeting moiety
(i) comprises a somatostatin analog that significantly binds to a polypeptide hormone receptor on a surface of a cell; or
(ii) comprises a natural or synthetic fragment of the natural ligand of a polypeptide receptor or is a molecule homologous to a portion of the natural ligand, and significantly binds to the polypeptide receptor on a surface of a cell wherein the polypeptide receptor is a neurotransmitter receptor or a hormone receptor or a growth factor receptor.

2. The drug delivery molecule of claim 1 wherein the receptor is a somatostatin type II receptor.

3. The drug delivery molecule of claim 2 wherein binding of the targeting moiety to a somatostatin type II receptor results in an uptake of the drug delivery molecule into the cell;
and wherein binding of the targeting moiety to a somatostatin type II receptor does not elicit a significant agonistic effect;
wherein a "significant agonist effect" is when binding elicits a measurable effect on the cell that is at least 25% of a measurable effect of the physiological ligand under comparable conditions.

4. The drug delivery molecule of claim 1 wherein the receptor has a natural ligand and the targeting moiety is a fragment of the natural ligand, or is a molecule homologous to a portion of the natural ligand.

5. The drug delivery molecule of claim 4 wherein the fragment of the natural ligand is synthetic.

6. The drug delivery molecule of any one of claims 1 to 5 wherein the targeting moiety comprises a cyclic peptide.

7. The drug delivery molecule of claim 1 wherein the targeting moiety comprises a molecule selected from the group consisting of: 
D-Trp — Lys
Phe                    Phe
7-Amino-heptanedioic acid
,
Lys
D-Trp          Abu
Phe                    Phe
2-Amino-heptanedioic acid
,
 
D-Phe — Cys ——————————— Phe
D-Trp
Thr — Cys — Aminopentanoic acid — Thr — Lys ,
 and
D-Phe-Cys-Phe-D-Trp-Orn-Abu-Cys-Thr.

8. The drug delivery molecule of claim 1 wherein the cell is a microvascular endothelial cell located proximal to a tumor.

9. The drug delivery molecule of claim 8 wherein the tumor is selected from the group consisting of a mammacarcinoma, an adenocarcinoma, a glioblastoma, a teratocarcinoma, a sarcoma, a lymphoma, and a neuroblastoma.

10. The drug delivery molecule of any one of claims 1 to 9 wherein the cell expresses the somatostatin type II receptor in a detectable amount and is a prostate carcinoma cell, or a lung carcinoma cell, or is a target cell from a tumour selected from the group consisting of a mammacarcinoma, an adenocarcinoma, a glioblastoma, a teratocarcinoma, a sarcoma, a lymphoma, and a neuroblastoma, and wherein the bioactive molecule comprises a drug.

11. The drug delivery molecule of any one of claims 1 to 9 wherein the bioactive molecule comprises one out of a short peptide sequence that is a substrate for an intracellular protease, a disulfide bond, a charge-complementary coupling, an ionic/coordinating interaction, a hydrogen bond, a hydrophobic interaction;
wherein the cell expresses the somatostatin type II receptor in a detectable amount and is a prostate carcinoma cell, or a lung carcinoma cell, or is a target cell from a tumour selected from the group consisting of a mammacarcinoma, an adenocarcinoma, a glioblastoma, a teratocarcinoma, a sarcoma, a lymphoma, and a neuroblastoma.

12. The drug delivery molecule of claim 10 wherein the drug comprises at least one of a molecule that hybridizes with a nucleic acid, and a nucleic acid that is expressed in a cell.

13. The drug delivery molecule of claim 10 wherein the drug comprises a molecule that interferes with a detoxification process in a cell, a molecule that inhibits replication of a cell, a molecule that inhibits an enzyme, a molecule that induces apoptosis, or a molecule that inhibits apoptosis.

14. The drug delivery molecule of any one of the preceding claims wherein the targeting moiety is coupled to the routing moiety but not to the bioactive molecule
or
wherein the targeting moiety is coupled to the bioactive molecule but not to the routing moiety.

15. The drug delivery molecule of any one of the preceding claims wherein the coupling between the targeting moiety and the at least one of the routing moiety and bioactive molecule is uncoupled when the drug delivery molecule is within the cell.

16. A drug delivery molecule of claim 1 having the structure: 
D-Trp — Lys
Phe
Phe
7-Amino-heptanedioic acid
KKLKKRKLIEENPKKKRKV
BAM
,
 
D-Phe-Cys-Phe-D-Trp-Orn-Abu-Cys-Thr
|
KKLKKRKLIEENPKKKRKV
|
BAM
,
 
Lys
D-Trp Abu
Phe Phe
2-Amino-heptanedioic acid
KKLKKRKLIEENPKKKRKV
BAM
 or, 
D-Phe — Cys — Phe
D-Trp
Lys
Thr — Cys — Aminopentanoic acid — Thr
KKLKKRKLIEENPKKKRKV
BAM
 wherein BAM is a bioactive molecule comprising a drug or a prodrug, said BAM further comprising one out of a short peptide sequence that is a substrate for an intracellular protease, a disulfide bond, a charge-complementary coupling, an ionic/coordinating interaction, a hydrogen bond, a hydrophobic interaction; or is a non-drug, non-prodrug reporter group that can be used for diagnostic purposes.

17. A compound suitable for use in a diagnostic environment, comprising a drug delivery molecule of claim 1 or claim 7, further comprising a reporter group wherein the reporter group is one out of a fluorophor, a chromophor, a hapten for a secondary antibody, a chromogenic substrate, a radionuclide, a metal detectable in scanning.

18. A drug delivery molecule comprising an antibody, a routing moiety, and a pharmacologically active substance,
wherein the antibody specifically binds to a somatostatin type II receptor, and is mono- or bispecific and conjugated with the pharmacologically active substance,
wherein the routing moiety is at least one out of the routing moieties of claim 1.

19. A compound specifically targeting an endothelial cell,
comprising a drug delivery molecule of claim 7 or of claim 18, wherein the compound specifically targeting the endothelial cell further comprises a nucleic acid; or
comprising a drug delivery molecule of claim 18 wherein the compound specifically targeting the endothelial cell further comprises at least one out of a cytokine or growth factor.

20. The drug delivery molecule of claim 18 wherein the pharmacologically active substance comprises a radionuclide or a thrombin.

21. An *ex vivo* method of selectively targeting an endothelial cell proximally located to an anomalous cell, comprising:
recognizing that the endothelial cell proximally located to the anomalous cell has a detectable amount of a somatostatin type II receptor, and that an endothelial cell not proximally located to the anomalous cell is devoid of the detectable amount of the somatostatin type II receptor; and
presenting the endothelial cell proximally located to the anomalous cell with a drug delivery molecule according to claim 2 that specifically binds to the somatostatin type II receptor.

22. The method of claim 21, wherein the anomalous cell is a neoplastic cell.

23. The method of claim 22, wherein the neoplastic cell is a mammacarcinoma cell, a prostate carcinoma cell, or a lung carcinoma cell.

24. The method of any one of claims 21 to 23, wherein the drug delivery molecule comprises a somatostatin, or a somatostatin analog.

25. A drug delivery molecule according to claim 2 that specifically binds to a somatostatin type II receptor for use in selectively targeting an endothelial cell proximally located to an anomalous cell which is a neoplastic cell, an ischemically stressed cell or a parenchymal cell of a tumour, in the treatment or diagnosis of the neoplastic cell, ischemically stressed cell or parenchymal cell of a tumour, in which the targeting comprises:
recognizing that the endothelial cell proximally located to the anomalous cell has a detectable amount of the somatostatin type II receptor, and that an endothelial cell not proximally located to the anomalous cell is devoid of the detectable amount of the somatostatin type II receptor; and
presenting the endothelial cell proximally located to the anomalous cell with the drug delivery molecule.

26. The drug delivery molecule of claim 25, wherein the anomalous cell is a neoplastic cell.

27. The drug delivery molecule of claim 26, wherein the neoplastic cell is a lymphoma, a sarcoma, an adenocarcinoma, a teratocarcinoma, a glioblastoma, a neuroblastoma, a mammacarcinoma cell, a prostate carcinoma cell, or a lung carcinoma cell.

28. The drug delivery molecule of claim 25, wherein the anomalous cell is an ischemically stressed cell.

29. The drug delivery molecule of claim 28, wherein the ischemically stressed cell is a cell within a layer of retinal pigment epithelia, a myocyte, a member of a plurality of cells that form a blood brain barrier, a neuron, or a transplanted heterologous cell.

30. The drug delivery molecule of any one of claims 25 to 29, wherein the drug delivery molecule comprises a somatostatin, or a somatostatin analog.

31. A drug delivery molecule according to claim 2 that specifically binds to a somatostatin type II receptor, for use in improving circulation, by a method comprising:
correlating a tissue having a reduced circulation with a detectable amount of the somatostatin type II receptor on an endothelial cell proximally located to the tissue having the reduced circulation; and
administering the drug delivery molecule to the endothelial cell wherein the drug delivery molecule stimulates a cell proliferation of the endothelial cell.

32. The drug delivery molecule of claim 31, wherein the reduced circulation is caused by a stenosis of a blood vessel.

33. The drug delivery molecule of claim 32, wherein the stenosis is in a location selected from the group consisting of a brain and a heart.

34. A drug delivery molecule according to claim 2 that specifically binds to a somatostatin type II receptor for use in improving vision by a method comprising:
correlating a tissue having a focus of macular degeneration with a detectable amount of the somatostatin type II receptor on an endothelial cell proximally located to the tissue having the focus of macular degeneration; and
administering the drug delivery molecule to the endothelial cell wherein the compound inhibits a cell proliferation of the endothelial cell.

35. A drug delivery molecule according to claim 2 that specifically binds to a somatostatin type II receptor, for use in improving a health condition associated with a neoplasm, whereby the health condition is dependent on the proliferation of anomalous cells in neoplastic tissue and endothelial cells proximal to the tissue having the neoplasm, the improving comprising:
correlating a tissue having a neoplasm with a detectable amount of the somatostatin type II receptor on an endothelial cell proximally located to the tissue having the neoplasm; and
administering the drug delivery molecule to the endothelial cell wherein the bioactive molecule inhibits a cell proliferation of the endothelial cell.

36. The drug delivery molecule of claim 35 wherein the neoplasm is a lymphoma, a sarcoma, an adenocarcinoma, or a teratocarcinoma or neuroblastoma, a mammacarcinoma, a prostate carcinoma or a lung cell carcinoma.

## Patentansprüche

1. Arzneimittelverabreichungsmolekül, umfassend:
eine Targetingeinheit, die signifikant an einen Rezeptor an einer Zelloberfläche bindet und wobei ein Binden der Targetingeinheit an den Rezeptor zu einer Aufnahme des Arzneimittelverabreichungsmoleküls in die Zelle führt;
eine Routingeinheit, die aus einer Kerntranslokationssignalsequenz, einem cytoplasmatischen Retentionssignal, einem endoplasmatischen Retikulumexportsignal oder einem mitochondrialen Translokationssignal ausgewählt ist; und
ein bioaktives Molekül, das an die Routingeinheit gekoppelt ist, wobei mindestens eines der Routingeinheit und des bioaktiven Moleküls an die Targetingeinheit gekoppelt ist;
und wobei das bioaktive Molekül (BAM) ein Arzneimittel oder ein Prodrug umfasst, wobei das BAM ferner eines von einer kurzen Peptidsequenz, die ein Substrat für eine intrazelluläre Protease ist, einer Disulfidbindung, einer Ladungsergänzungskopplung, einer ionischen/koordinierenden Interaktion, einer Wasserstoffbindung, einer hydrophoben Interaktion umfasst; oder das BAM ist eine Nicht-Arzneimittel-, Nicht-Prodrug-Reportergruppe, die zu diagnostischen Zwecken verwendet werden kann;
und wobei die Targetingeinheit
(i) ein Somatostatinanalogon umfasst, das signifikant an einen Polypeptidhormonrezeptor an einer Zelloberfläche bindet; oder
(ii) ein natürliches oder synthetisches Fragment des natürlichen Liganden eines Polypeptidrezeptors umfasst oder ein Molekül ist, das homolog zu einem Teil des natürlichen Liganden ist, und signifikant an den Polypeptidrezeptor an einer Zelloberfläche bindet, wobei der Polypeptidrezeptor ein Neurotransmitterrezeptor oder ein Hormonrezeptor oder ein Wachstumsfaktorrezeptor ist.

2. Arzneimittelverabreichungsmolekül nach Anspruch 1, wobei der Rezeptor ein Somatostatinrezeptor des Typs II ist.

3. Arzneimittelverabreichungsmolekül nach Anspruch 2, wobei ein Binden der Targetingeinheit an einen Somatostatinrezeptor des Typs II zu einer Aufnahme des Arzneimittelverabreichungsmoleküls in die Zelle führt;
und wobei ein Binden der Targetingeinheit an einen Somatostatinrezeptor des Typs II keine signifikante antagonistische Wirkung hervorruft;
wobei eine "signifikante antagonistische Wirkung" vorliegt, wenn ein Binden eine messbare Wirkung auf die Zelle hervorruft, die mindestens 25 % einer messbaren Wirkung auf den physiologischen Liganden unter vergleichbaren Bedingungen ist.

4. Arzneimittelverabreichungsmolekül nach Anspruch 1, wobei der Rezeptor einen natürlichen Liganden hat und die Targetingeinheit ein Fragment des natürlichen Liganden ist oder ein Molekül ist, das homolog zu einem Teil des natürlichen Liganden ist.

5. Arzneimittelverabreichungsmolekül nach Anspruch 4, wobei das Fragment des natürlichen Liganden synthetisch ist.

6. Arzneimittelverabreichungsmolekül nach einem der Ansprüche 1 bis 5, wobei die Targetingeinheit ein Cyclopeptid umfasst.

7. Arzneimittelverabreichungsmolekül nach Anspruch 1, wobei die Targetingeinheit ein Molekül umfasst, das aus der Gruppe, bestehend aus: 
D-Trp — Lys
Phe                    Phe
7-Amino-Heptandisäure
,
 
Lys
D-Trp Abu
Phe Phe
2-Amino-Heptandisäure
,
 
D-Phe — Cys ——————————————— Phe
D-Trp
Lys
Thr — Cys — Aminopentansäure — Thr
 und
D-Phe-Cys-Phe-D-Trp-Om-Abu-Cys-Thr,
ausgewählt ist.

8. Arzneimittelverabreichungsmolekül nach Anspruch 1, wobei die Zelle eine mikrovaskuläre Endothelzelle ist, die sich nahe an einem Tumor befindet.

9. Arzneimittelverabreichungsmolekül nach Anspruch 8, wobei der Tumor aus der Gruppe, bestehend aus einem Mammakarzinom, einem Adenokarzinom, einem Glioblastom, einem Teratokarzinom, einem Sarkom, einem Lymphom und einem Neuroblastom, ausgewählt ist.

10. Arzneimittelverabreichungsmolekül nach einem der Ansprüche 1 bis 9, wobei die Zelle den Somatostatinrezeptor des Typs II in einer nachweisbaren Menge exprimiert und eine Prostatakarzinomzelle oder eine Lungenkarzinomzelle oder eine Zielzelle von einem Tumor ist, der aus der Gruppe, bestehend aus einem Mammakarzinom, einem Adenokarzinom, einem Glioblastom, einem Teratokarzinom, einem Sakrom, einem Lymphom und einem Neuroblastom, ausgewählt ist und wobei das bioaktive Molekül ein Arzneimittel umfasst.

11. Arzneimittelverabreichungsmolekül nach einem der Ansprüche 1 bis 9, wobei das bioaktive Molekül eines von einer kurzen Peptidsequenz, die ein Substrat für eine intrazelluläre Protease ist, einer Disulfidbindung, einer Ladungsergänzungskopplung, einer ionischen/koordinierenden Interaktion, einer Wasserstoffbindung, einer hydrophoben Interaktion umfasst;
wobei die Zelle den Somatostatinrezeptor des Typs II in einer nachweisbaren Menge exprimiert und eine Prostatakarzinomzelle oder eine Lungenkarzinomzelle oder eine Zielzelle von einem Tumor ist, der aus der Gruppe, bestehend aus einem Mammakarzinom, einem Adenokarzinom, einem Glioblastom, einem Teratokarzinom, einem Sakrom, einem Lymphom und einem Neuroblastom, ausgewählt ist.

12. Arzneimittelverabreichungsmolekül nach Anspruch 10, wobei das Arzneimittel mindestens eines von einem Molekül, das mit einer Nukleinsäure hybridisiert, und einer Nukleinsäure, die in einer Zelle exprimiert ist, umfasst.

13. Arzneimittelverabreichungsmolekül nach Anspruch 10, wobei das Arzneimittel ein Molekül, das einen Entgiftungprozess in einer Zelle beeinträchtigt, ein Molekül, das eine Replikation einer Zelle hemmt, ein Molekül, das ein Enzym hemmt, ein Molekül, das Apoptose induziert, oder ein Molekül, das Apoptose hemmt, umfasst.

14. Arzneimittelverabreichungsmolekül nach einem der vorangehenden Ansprüche, wobei die Targetingeinheit an die Routingeinheit gekoppelt ist, aber nicht an das bioaktive Molekül,
oder
wobei die Targetingeinheit an das bioaktive Molekül gekoppelt ist, aber nicht an die Routingeinheit.

15. Arzneimittelverabreichungsmolekül nach einem der vorangehenden Ansprüche, wobei die Kopplung zwischen der Targetingeinheit und dem mindestens einen der Routingeinheit und des bioaktiven Moleküls entkoppelt ist, wenn das Arzneimittelverabreichungsmolekül sich in der Zelle befindet.

16. Arzneimittelverabreichungsmolekül nach Anspruch 1 mit der Struktur: 
D-Trp — Lys
Phe
Phe
7-Amino-Heptandisäure
KKLKKRKLIEENPKKKRKV
BAM
,
 
D-Phe-Cys-Phe-D-Trp-Orn-Abu-Cys-Thr
|
KKLKKRKLIEENPKKKRKV
|
BAM
,
 
Lys
D-Trp Abu
Phe Phe
2-Amino-Heptandisäure
KKLKKRKLIEENPKKKRKV
BAM
 oder 
D-Phe — Cys — Phe
D-Trp
Cys
Lys
Thr — Cys — Aminopentansäure — Thr
KKLKKRKLIEENPKKKRKV
BAM
 wobei BAM ein bioaktives Molekül ist, das ein Arzneimittel oder ein Prodrug umfasst, wobei das BAM ferner eines von einer kurzen Peptidsequenz, die ein Substrat für eine intrazelluläre Protease ist, einer Disulfidbindung, einer Ladungsergänzungskopplung, einer ionischen/koordinierenden Interaktion, einer Wasserstoffbindung, einer hydrophoben Interaktion umfasst; oder eine Nicht-Arzneimittel-, Nicht-Prodrug-Reportergruppe ist, die zu diagnostischen Zwecken verwendet werden kann;

17. Verbindung, die zur Verwendung in einer diagnostischen Umgebung geeignet ist, umfassend ein Arzneimittelverabreichungsmolekül nach Anspruch 1 oder Anspruch 7, ferner umfassend eine Reportergruppe, wobei die Reportergruppe eines von einem Fluorphor, einem Chromophor, einem Hapten für einen sekundären Antikörper, einem chromogenen Substrat, einem Radionuklid, einem beim Scannen erfassbaren Metall ist.

18. Arzneimittelverabreichungsmolekül, umfassend einen Antikörper, eine Routingeinheit und eine pharmakologisch aktive Substanz,
wobei der Antikörper spezifisch an einen Somatostatinrezeptor des Typs II bindet und mono- oder bispezifisch ist und mit der pharmakologisch aktiven Substanz konjugiert ist,
wobei die Routingeinheit mindestens eine der Routingeinheiten nach Anspruch 1 ist.

19. Verbindung, die spezifisch eine Endothelzelle anzielt,
umfassend ein Arzneimittelverabreichungsmolekül nach Anspruch 7 oder Anspruch 18, wobei die Verbindung, die spezifisch die Endothelzelle anzielt, ferner eine Nukleinsäure umfasst; oder
umfassend ein Arzneimittelverabreichungsmolekül nach Anspruch 18, wobei die Verbindung, die spezifisch die Endothelzelle anzielt, ferner mindestens eines eines Cytokins oder Wachstumsfaktors umfasst.

20. Arzneimittelverabreichungsmolekül nach Anspruch 18, wobei die pharmakologisch aktive Substanz ein Radionuklid oder ein Thrombin umfasst.

21. *Ex vivo*-Verfahren zum selektiven Anzielen einer Endothelzelle nahe an einer anormalen Zelle, umfassend:
Erkennen, dass die Endothelzelle nahe an der anormalen Zelle eine nachweisbare Menge eines Somatostatinrezeptors des Typs II hat und dass eine von der anormalen Zelle entfernte Endothelzelle keine nachweisbare Menge des Somatostatinrezeptors des Typs II aufweist; und
Überreichen des Arzneimittelverabreichungsmoleküls nach Anspruch 2, das spezifisch an den Somatostatinrezeptor des Typs II bindet, an die Endothelzelle nahe an der anormalen Zelle.

22. Verfahren nach Anspruch 21, wobei die anormale Zelle eine neoplastische Zelle ist.

23. Verfahren nach Anspruch 22, wobei die neoplastische Zelle eine Mammakarzinomzelle, eine Prostatakarzinomzelle oder eine Lungenkarzinomzelle ist.

24. Verfahren nach einem der Ansprüche 21 bis 23, wobei das Arzneimittelverabreichungsmolekül ein Somatostatin oder ein Somatostatinanalogon umfasst.

25. Arzneimittelverabreichungsmolekül nach Anspruch 2, das spezifisch an einen Somatostatinrezeptor des Typs II bindet, zur Verwendung bei einem selektiven Anzielen einer Endothelzelle nahe an einer anormalen Zelle, die eine neoplastische Zelle, eine ischämisch beanspruchte Zelle oder eine parenchymale Zelle eines Tumors ist, bei der Behandlung oder Diagnose der neoplastischen Zelle, ischämisch beanspruchten Zelle oder parenchymalen Zelle eines Tumors, bei der das Anzielen Folgendes umfasst:
Erkennen, dass die Endothelzelle nahe an der anormalen Zelle eine nachweisbare Menge eines Somatostatinrezeptors des Typs II hat und dass eine von der anormalen Zelle entfernte Endothelzelle keine nachweisbare Menge des Somatostatinrezeptors des Typs II aufweist; und
Überreichen des Arzneimittelverabreichungsmoleküls an die Endothelzelle nahe an der anormalen Zelle.

26. Arzneimittelverabreichungsmolekül nach Anspruch 25, wobei die anormale Zelle eine neoplastische Zelle ist.

27. Arzneimittelverabreichungsmolekül nach Anspruch 26, wobei die neoplastische Zelle ein Lymphom, ein Sarkom, ein Ardenokarzinom, ein Teratokarzinom, ein Glioblastom, ein Neuroblastom, eine Mammakarzinomzelle, eine Prostatakarzinomzelle oder eine Lungenkarzinomzelle ist.

28. Arzneimittelverabreichungsmolekül nach Anspruch 25, wobei die anormale Zelle eine ischämisch beanspruchte Zelle ist.

29. Arzneimittelverabreichungsmolekül nach Anspruch 28, wobei die ischämisch beanspruchte Zelle eine Zelle in einer Schicht retinaler Pigmentepithele, ein Myocyt, ein Teil einer Vielzahl von Zellen, die eine Blutgehirnschranke bilden, ein Neuron oder eine transplantierte heterologe Zelle ist.

30. Arzneimittelverabreichungsmolekül nach einem der Ansprüche 25 bis 29, wobei das Arzneimittelverabreichungsmolekül ein Somatostatin oder ein Somatostatinanalogon umfasst.

31. Arzneimittelverabreichungsmolekül nach Anspruch 2, das spezifisch an einen Somatostatinrezeptor des Typs II bindet, zur Verwendung bei einer Verbesserung der Durchblutung durch ein Verfahren, das Folgendes umfasst:
Korrelieren eines Gewebes mit verminderter Durchblutung mit einer nachweisbaren Menge des Somatostatinrezeptors des Typs II an einer Endothelzelle, die sich nahe an dem Gewebe mit verminderter Durchblutung befindet; und
Verabreichen des Arzneimittelverabreichungsmoleküls an die Endothelzelle, wobei das Arzneimittelverabreichungsmolekül eine Zellproliferation der Endothelzelle anregt.

32. Arzneimittelverabreichungsmolekül nach Anspruch 31, wobei die verminderte Durchblutung durch eine Stenose eines Blutgefäßes verursacht wird.

33. Arzneimittelverabreichungsmolekül nach Anspruch 32, wobei sich die Stenose an einer Stelle befindet, die aus der Gruppe, bestehend aus einem Gehirn und einem Herzen, ausgewählt ist.

34. Arzneimittelverabreichungsmolekül nach Anspruch 2, das spezifisch an einen Somatostatinrezeptor des Typs II bindet, zur Verwendung bei einer Verbesserung der Sehkraft durch ein Verfahren, das Folgendes umfasst:
Korrelieren eines Gewebes mit Makuladegenerationsfokus mit einer nachweisbaren Menge des Somatostatinrezeptors des Typs II an einer Endothelzelle, die sich nahe an dem Gewebe mit Makuladegenerationsfokus befindet; und
Verabreichen des Arzneimittelverabreichungsmoleküls an die Endothelzelle, wobei die Verbindung eine Zellproliferation der Endothelzelle hemmt.

35. Arzneimittelverabreichungsmolekül nach Anspruch 2, das spezifisch an einen Somatostatinrezeptor des Typs II bindet, zur Verwendung bei einem Verbessern eines mit einem Neoplasma assoziierten Gesundheitszustandes, wobei der Gesundheitszustand von der Proliferation von anormalen Zellen in neoplastischem Gewebe und von Endothelzellen nahe dem Gewebe mit dem Neoplasma abhängt, das Verbessern umfassend:
Korrelieren eines Gewebes mit einem Neoplasma mit einer nachweisbaren Menge des Somatostatinrezeptors des Typs II an einer Endothelzelle, die sich nahe an dem Gewebe mit dem Neoplasma befindet; und
Verabreichen des Arzneimittelverabreichungsmoleküls an die Endothelzelle, wobei das bioaktive Molekül eine Zellproliferation der Endothelzelle hemmt.

36. Arzneimittelverabreichungsmolekül nach Anspruch 35, wobei das Neoplasma ein Lymphom, ein Sarkom, ein Ardenokarzinom, ein Teratokarzinom oder Neuroblastom, ein Mammakarzinom, ein Prostatakarzinom oder ein Lungenzellkarzinom ist.

## Revendications

1. Molécule d'administration de médicament, comprenant :
un groupe fonctionnel de ciblage qui se lie de manière significative à un récepteur sur la surface d'une cellule, et la liaison du groupe fonctionnel de ciblage au récepteur entraînant une absorption de la molécule d'administration de médicament dans la cellule ;
un groupe fonctionnel de cheminement choisi parmi une séquence de signaux de translocation nucléaire, un signal de rétention cytoplasmique, un signal d'export de réticule endoplasmique ou un signal de translocation mitochondriale ; et
une molécule bioactive, couplée au groupe fonctionnel de cheminement, le groupe fonctionnel de cheminement et/ou la molécule bioactive étant couplés au groupe fonctionnel de ciblage ;
et dans lequel la molécule bioactive (BAM) comprenant un médicament ou un pro-médicament, ladite BAM comprenant en outre une courte séquence peptidique qui est un substrat pour une protéase intracellulaire, une liaison disulfure, un couplage complémentaire de charge, une interaction ionique/de coordination, une liaison hydrogène, une interaction hydrophobe ; ou la BAM est un groupe rapporteur non médicamenteux, non pro-médicamenteux qui peut être utilisé à des fins diagnostiques ;
et dans lequel le groupe fonctionnel de ciblage
(i) comprend un analogue de la somatostatine qui se lie de manière significative à un récepteur d'hormone polypeptidique sur la surface d'une cellule ; ou
(ii) comprend un fragment naturel ou synthétique du ligand naturel d'un récepteur de polypeptide ou est une molécule homologue à une partie du ligand naturel, et se lie de manière significative au récepteur de polypeptide sur la surface d'une cellule, le récepteur de polypeptide étant un récepteur de neurotransmetteur ou un récepteur d'hormone ou un récepteur de facteur de croissance.

2. Molécule d'administration de médicament selon la revendication 1, dans laquelle le récepteur est un récepteur de somatostatine de type II.

3. Molécule d'administration de médicament selon la revendication 2, dans laquelle la liaison du groupe fonctionnel de ciblage à un récepteur de somatostatine de type II entraîne une absorption de la molécule d'administration de médicament dans la cellule ;
et dans laquelle la liaison du groupe fonctionnel de ciblage à un récepteur de somatostatine de type II ne déclenche pas un effet agoniste important ;
un « effet agoniste important » étant lorsqu'une liaison déclenche un effet mesurable sur la cellule qui est à au moins 25 % d'un effet mesurable du ligand physiologique dans des conditions comparables.

4. Molécule d'administration de médicament selon la revendication 1, dans laquelle le récepteur présente un ligand naturel et le groupe fonctionnel de ciblage est un fragment du ligand naturel ou est une molécule homologue à une partie du ligand naturel.

5. Molécule d'administration de médicament selon la revendication 4, dans laquelle le fragment du ligand naturel est synthétique.

6. Molécule d'administration de médicament selon l'une quelconque des revendications 1 à 5, le groupe fonctionnel de ciblage comprenant un peptide cyclique.

7. Molécule d'administration de médicament selon la revendication 1, dans laquelle le groupe fonctionnel de ciblage comprend une molécule choisie dans le groupe constitué par : 
D-Trp — Lys
Phe                    Phe
acide 7-amino-heptanedioique
,
 
Lys
D-Trp Abu
Phe Phe
acide 2-amino-heptanedioique
 
D-Phe — Cys ——————————————— Phe
D-Trp
Thr — Cys — acide aminopentanoique — Thr — Lys
,
 et
D-Phe-Cys-Phe-D-Trp-Orn-Abu-Cys-Thr.

8. Molécule d'administration de médicament selon la revendication 1, dans laquelle la cellule est une cellule endothéliale micro-vasculaire située à proximité d'une tumeur.

9. Molécule d'administration de médicament selon la revendication 8, dans laquelle la tumeur est choisie dans le groupe constitué par un mammacarcinome, un adénocarcinome, un glioblastome, un tératocarcinome, un sarcome, un lymphome et un neuroblastome.

10. Molécule d'administration de médicament selon l'une quelconque des revendications 1 à 9, dans laquelle la cellule exprime le récepteur de somatostatine de type II dans une quantité détectable et est une cellule de carcinome de la prostate ou une cellule de carcinome du poumon ou est une cellule cible provenant d'une tumeur choisie dans le groupe constitué par un mammacarcinome, un adénocarcinome, un glioblastome, un tératocarcinome, un sarcome, un lymphome et un neuroblastome, et la molécule bioactive comprend un médicament.

11. Molécule d'administration de médicament selon l'une quelconque des revendications 1 à 9, dans laquelle la molécule bioactive comprend une courte séquence peptidique qui est un substrat pour une protéase intracellulaire, une liaison disulfure, un couplage complémentaire de charge, une interaction ionique / de coordination, une liaison hydrogène ou une interaction hydrophobe ;
dans laquelle la cellule exprime le récepteur de somatostatine de type II dans une quantité détectable et est une cellule de carcinome de la prostate ou une cellule de carcinome du poumon ou est une cellule cible provenant d'une tumeur choisie dans le groupe constitué par un mammacarcinome, un adénocarcinome, un glioblastome, un tératocarcinome, un sarcome, un lymphome et un neuroblastome.

12. Molécule d'administration de médicament selon la revendication 10, dans laquelle le médicament comprend une molécule qui s'hybride avec un acide nucléique et un acide nucléique qui est exprimé dans une cellule.

13. Molécule d'administration de médicament selon la revendication 10, dans laquelle le médicament comprend une molécule qui interfère avec un processus de détoxification dans une cellule, une molécule qui inhibe la réplication d'une cellule, une molécule qui inhibe une enzyme, une molécule qui induit l'apoptose ou une molécule qui inhibe l'apoptose.

14. Molécule d'administration de médicament selon l'une quelconque des revendications précédentes, dans laquelle le groupe fonctionnel de ciblage est couplé au groupe fonctionnel de cheminement mais pas à la molécule bioactive
ou
dans laquelle le groupe fonctionnel de ciblage est couplé à la molécule bioactive mais pas au groupe fonctionnel de cheminement.

15. Molécule d'administration de médicament selon l'une quelconque des revendications précédentes, dans laquelle le couplage entre le groupe fonctionnel de ciblage et ledit groupe fonctionnel de cheminement et/ou ladite molécule bioactive est découplé lorsque la molécule d'administration de médicament se trouve à l'intérieur de la cellule.

16. Molécule d'administration de médicament selon la revendication 1 présentant la structure : 
D-Trp — Lys
Phe
Phe
acide 7-amino-heptanedioique
KKLKKRKLIEENPKKKRKV
BAM
 
D-Phe-Cys-Phe-D-Trp-Orn-Abu-Cys-Thr
|
KKLKKRKLIEENPKKKRKV
|
BAM
 
Lys
D-Trp Abu
Phe Phe
acide 2-amino-heptanedioique
KKLKKRKLIEENPKKKRKV
BAM
 
D-Phe — Cys ——————————— Phe
D-Trp
Lys
Thr — Cys — acide aminopentanoique — Thr
KKLKKRKLIEENPKKKRKV
BAM
 où la BAM une molécule bioactive comprenant un médicament ou un pro-médicament, ladite BAM comprenant en outre une courte séquence peptidique qui est un substrat pour une protéase intracellulaire, une liaison disulfure, un couplage complémentaire de charge, une interaction ionique / de coordination, une liaison hydrogène, une interaction hydrophobe ; ou est un groupe rapporteur non médicamenteux, non pro-médicamenteux qui peut être utilisé à des fins diagnostiques.

17. Composé destiné à être utilisé dans un environnement de diagnostic, comprenant une molécule d'administration de médicament selon la revendication 1 ou la revendication 7, comprenant en outre un groupe rapporteur, le groupe rapporteur étant un fluorophore, un chromophore, un haptène pour un anticorps secondaire, un substrat chromogène, un radionucléide ou un métal détectable lors d'un balayage.

18. Molécule d'administration de médicament comprenant un anticorps, un groupe fonctionnel de cheminement et une substance pharmacologiquement active,
dans laquelle l'anticorps se lie spécifiquement à un récepteur de somatostatine de type II et est mono- ou bispécifique et conjugué à la substance pharmacologiquement active,
dans laquelle le groupe fonctionnel de cheminement est au moins un des groupes fonctionnels de cheminement selon la revendication 1.

19. Composé ciblant spécifiquement une cellule endothéliale,
comprenant une molécule d'administration de médicament selon la revendication 7 ou la revendication 18, le composé ciblant spécifiquement la cellule endothéliale comprenant en outre un acide nucléique ; ou
comprenant une molécule d'administration de médicament selon la revendication 18, le composé ciblant spécifiquement la cellule endothéliale comprenant en outre une cytokine et/ou un facteur de croissance.

20. Molécule d'administration de médicament selon la revendication 18, dans laquelle la substance pharmacologiquement active comprend un radionucléide ou une thrombine.

21. Procédé *ex vivo* consistant à cibler de manière sélective une cellule endothéliale située à proximité d'une cellule anormale, comprenant :
la reconnaissance que la cellule endothéliale située à proximité de la cellule anormale présente une quantité détectable d'un récepteur de somatostatine de type II, et qu'une cellule endothéliale non située à proximité de la cellule anormale est dépourvue de la quantité détectable du récepteur de somatostatine de type II ; et
la présentation à la cellule endothéliale située à proximité de la cellule anormale d'une molécule d'administration de médicament selon la revendication 2 qui se lie spécifiquement au récepteur de somatostatine de type II.

22. Procédé selon la revendication 21, dans lequel la cellule anormale est une cellule néoplasique.

23. Procédé selon la revendication 22, dans lequel la cellule néoplasique est une cellule de mammacarcinome, une cellule de carcinome de la prostate ou une cellule de carcinome du poumon.

24. Procédé selon l'une quelconque des revendications 21 à 23, dans lequel la molécule d'administration de médicament comprend une somatostatine ou un analogue de somatostatine.

25. Molécule d'administration de médicament selon la revendication 2 qui se lie spécifiquement à un récepteur de somatostatine de type II destiné à une utilisation dans le ciblage sélectif d'une cellule endothéliale située à proximité d'une cellule anormale qui est une cellule néoplasique, une cellule stressée par une ischémie ou une cellule parenchymateuse d'une tumeur, dans le traitement ou le diagnostic de la cellule néoplasique, de la cellule stressée par une ischémie ou de la cellule parenchymateuse d'une tumeur, le ciblage comprenant :
la reconnaissance que la cellule endothéliale située à proximité de la cellule anormale présente une quantité détectable du récepteur de somatostatine de type II, et qu'une cellule endothéliale non située à proximité de la cellule anormale est dépourvue de la quantité détectable du récepteur de somatostatine de type II ; et
la présentation à la cellule endothéliale située à proximité de la cellule anormale de la molécule d'administration de médicament.

26. Molécule d'administration de médicament selon la revendication 25, dans laquelle la cellule anormale est une cellule néoplasique.

27. Molécule d'administration de médicament selon la revendication 26, dans laquelle la cellule néoplasique est un lymphome, un sarcome, un adénocarcinome, un tératocarcinome, un glioblastome, un neuroblastome, une cellule de mammacarcinome, une cellule de carcinome de la prostate ou une cellule de carcinome du poumon.

28. Molécule d'administration de médicament selon la revendication 25, dans laquelle la cellule anormale est une cellule stressée par une ischémie.

29. Molécule d'administration de médicament selon la revendication 28, dans laquelle la cellule stressée par une ischémie est une cellule à l'intérieur d'une couche d'épithélium pigmentaire de la rétine, un myocyte, un élément d'une pluralité de cellules qui forment une barrière hématoencéphalique, un neurone ou une cellule hétérologue transplantée.

30. Molécule d'administration de médicament selon l'une quelconque des revendications 25 à 29, la molécule d'administration de médicament comprenant une somatostatine ou un analogue de somatostatine.

31. Molécule d'administration de médicament selon la revendication 2 qui se lie spécifiquement à un récepteur de somatostatine de type II, en vue d'une utilisation dans l'amélioration de la circulation, par un procédé comprenant :
la corrélation d'un tissu présentant une circulation réduite avec une quantité détectable du récepteur de somatostatine de type II sur une cellule endothéliale située à proximité du tissu présentant la circulation réduite ; et
l'administration de la molécule d'administration de médicament à la cellule endothéliale, la molécule d'administration de médicament stimulant une prolifération cellulaire de la cellule endothéliale.

32. Molécule d'administration de médicament selon la revendication 31, dans laquelle la circulation réduite est provoquée par la sténose d'un vaisseau sanguin.

33. Molécule d'administration de médicament selon la revendication 32, dans laquelle la sténose se trouve dans un emplacement choisi dans le groupe constitué par un cerveau et un coeur.

34. Molécule d'administration de médicament selon la revendication 2 qui se lie spécifiquement à un récepteur de somatostatine de type II en vue d'une utilisation dans l'amélioration de la vue par un procédé comprenant :
la corrélation d'un tissu faisant l'objet de dégénérescence maculaire avec une quantité détectable du récepteur de somatostatine de type II sur une cellule endothéliale située à proximité du tissu faisant l'objet de dégénérescence maculaire ; et
l'administration de la molécule d'administration de médicament à la cellule endothéliale, le composé inhibant une prolifération cellulaire de la cellule endothéliale.

35. Molécule d'administration de médicament selon la revendication 2 qui se lie spécifiquement à un récepteur de somatostatine de type II, en vue d'une utilisation dans l'amélioration d'un problème de santé associé à un néoplasme, moyennant quoi le problème de santé est dépendant de la prolifération de cellules anormales dans un tissu néoplasique et des cellules endothéliales à proximité du tissu présentant le néoplasme, l'amélioration comprenant :
la corrélation d'un tissu présentant un néoplasme avec une quantité détectable du récepteur de somatostatine de type II sur une cellule endothéliale située à proximité du tissu présentant le néoplasme ; et
l'administration de la molécule d'administration de médicament à la cellule endothéliale, la molécule bioactive inhibant une prolifération cellulaire de la cellule endothéliale.

36. Molécule d'administration de médicament selon la revendication 35, dans laquelle le néoplasme est un lymphome, un sarcome, un adénocarcinome, un tératocarcinome ou un neuroblastome, un mammacarcinome, un carcinome de la prostate ou un carcinome de cellule du poumon.
